# EUROPEAN PATENT APPLICATION

(11) **EP 4 306 134 A1**
(43) Date of publication of application: **17.01.2024**
(21) Application number: 23184597.5
(22) Date of filing: 11.07.2023
(51) Int. Cl.: A61L 2/00, A61L 2/20, A61L 2/24, A61L 2/18

(54) **DEVICE FOR SANITIZING UPPER LIMBS**

(30) Priority: 14.07.2022 IT 202200014800
(71) Applicant: Marzella, Michele Giuseppe Renato, 66054 Vasto (CH) (IT); Tecnoline S.p.A., 41033 Concordia Sulla Secchia, MO (IT)
(72) Inventor: MARZELLA, MICHELE, 66054 VASTO (IT); PROVASI, STEFANO, 41033 CONCORDIA SULLA SECCHIA (IT)
(74) Representative: Conversano, Gabriele

(57) **Abstract**

Device for hands sanitization (1) comprising at least a compartment (10), with dimensions and shape suitable to receive the hands and at least a portion of the respective forearms of an operator, said at least one compartment (10) being provided with a couple of openings (11) each configured for the introduction of a hand or forearm, and provided in a membrane (12) of elastomeric material applied on the wall of said at least one compartment (10); means for ozone production (2), configured to generate an ozone flow to be introduced inside said at least one compartment (10); an air discharge duct (15) from inside said at least one compartment (10) outwards, characterized in that it comprises further at each of said openings (11), a respective lid (6) configured to close said opening (11) and to define a volume (116) contained between the outer portion of said membrane (12) and said lid (6) .

## Description

The present invention relates to a device for upper limbs sanitization, optimized for operating rooms and generally where it is needed to guarantee a perfect sanitization of an operator's hands and to trace reliably the sanitization occurred.

### State of the art

At the state of the art, there are known many examples of sanitization devices by means of ozone, both for objects and human body parts.

An example is described in the Italian Patent Application 102020000019804. In this document, it is described a sanitization device by means of ozone, suitable for hands sanitization as well, comprising a compartment for containing ozone, provided with an opening through which hands or an object to be sanitized can be introduced. The device is also provided with means for ozone production, means for the generation of an air flow to be sent towards the object to be sanitized, and means for converting ozone in oxygen by means of a catalyzer, in order to reduce the quantity of ozone released in ambient air outside the device during usage.

Other examples of similar devices are also known.

US20100266446 describes a device for hands disinfection having a first compartment provided with an inlet allowing the user hands and forearms introduction and with an ozone generator for hands sanitization. EP3858389 describes an apparatus for hands disinfection comprising a housing containing a user's hands receiving chamber, inside which an ozone source is provided. The device is configured to flux ozone on the hands introduced in the receiving chamber. AU633646 describes a medical equipment comprising: a compartment configured to receive hermetically, through an opening, the interested body parts; an ozonizer; a mechanism for the introduction of an ozonized air flow inside the hermetic body; a gas discharge mechanism to discharge ozone gas from the hermetic body while keeping its inner pressure. CN105902380 describes a therapeutic apparatus for dermatophytosis treatment comprising a shell provided with openings for hands introduction and with an ozone generator inside the shell itself.

### Technical problem

Even if all these documents describe devices that can be used for hands disinfection, they have nevertheless some drawbacks which impede their usage in some contexts, such for example the operating rooms.

In particular, none of the described devices is provided with means for automatic disinfection of the device itself, which prevent the user from contaminating his own hands or forearms while using the device. Moreover, none of the known devices is configured to avoid any contact of the user hands with the device during all its actuation steps. Yet, none of the devices at the state of the art is configured to trace reliably the correct execution of the hands sterilization procedure by an operator.

Yet, none of the known devices allows to reach all the just described advantages and to couple the mechanical action of washing with water to the disinfection by means of ozone.

### Aim of the invention

Therefore, aim of the present invention is to provide a device for hands sanitization which overcomes the limits linked to the devices known at the state of the art, which is provided with means for automatic disinfection of the device itself, which is configured to avoid any contact of the user hands with the device during its functioning and which allows to trace reliably the correct execution of the hands sterilization procedure by an operator. Furthermore, the device according to the invention allows to couple the mechanical action of washing with water to the sanitization by means of ozone.

### Brief description of the invention

The invention realizes the prefixed aims since it is a device for hands sanitization (1) comprising: at least a compartment (10), with dimensions and shape suitable to receive the hands and at least a portion of the respective forearms of an operator, said at least one compartment (10) being provided with a couple of openings (11) each configured for the introduction of a hand or forearm and provided in a membrane (12) of elastomeric material applied on the wall of said at least one compartment (10); means for ozone production (2), configured to generate an ozone flow to be introduced inside said at least one compartment (10); an air discharge duct (15) from inside said at least one compartment (10) outwards, characterized in that it comprises further, at each of said openings (11), a respective lid (6) configured to close said opening (11) and to define a volume (116) contained between the outer portion of said membrane (12) and said lid (6).

### Detailed description of the invention

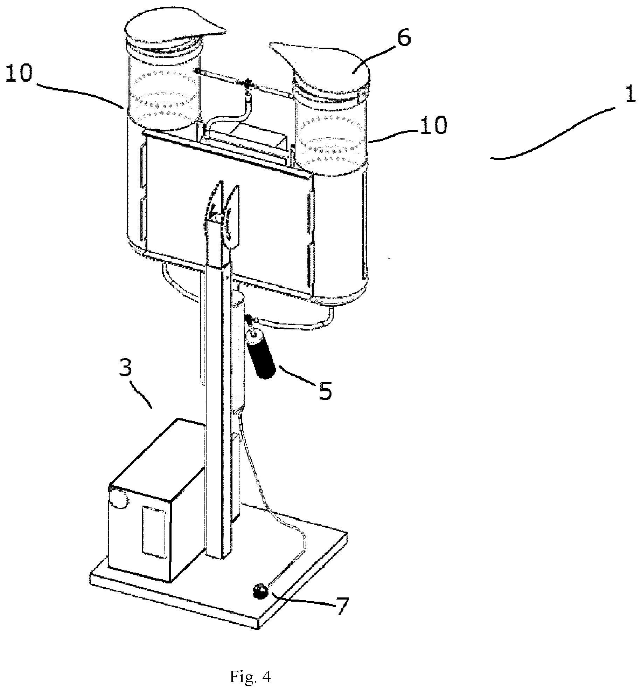

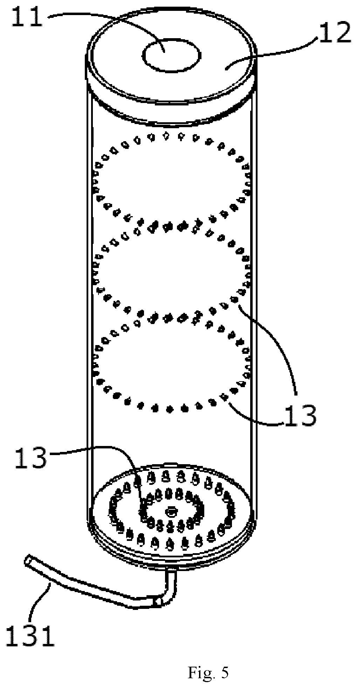

The invention will be described in the following with reference to the appended figures 1 to 6.
Figure 1 shows a front view of the device according to the invention in a preferred embodiment.
Figures 2 shows a section view of a compartment for one hand and forearm washing, shown in axonometric view in figure 5.
Figure 3 shows a particular of an automatic sterilization system of the device.
Figure 4 shows an axonometric view of the device according to the invention, in a preferred embodiment.
Figure 6 shows a view of an embodiment of the device with double membrane.

### Detailed description of the invention

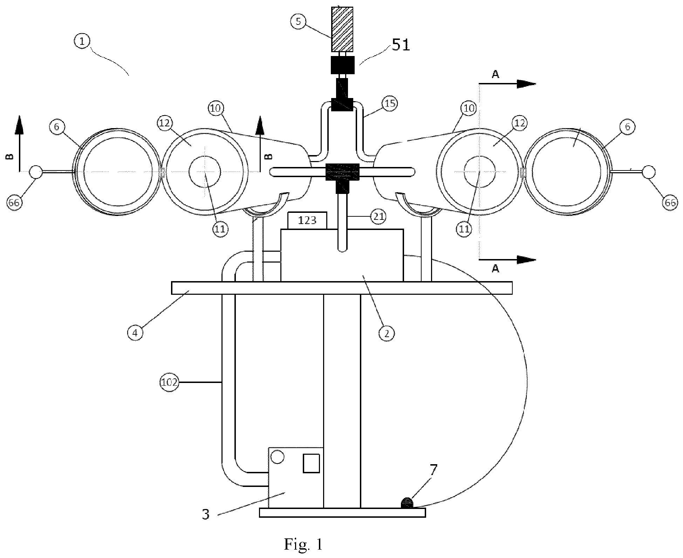

With reference to the appended figure 1, the device (1) shown comprises at least a compartment (10), with dimensions and shape suitable to receive an operator's hands and forearms. Said compartment (10) is provided with a couple of openings (11), each configured for a hand and forearm introduction. Said openings (11) have preferably circular shape and are provided in a membrane (12) applied on the wall of said compartment (10) and realized in elastomeric material - for example silicone - so to provide sealing to the operator's forearm once this is introduced, and to adapt to the various sizes of different hands and forearms.

According to a preferred embodiment, the device comprises a couple of compartments (10), with dimensions and shape suitable to receive each a hand and respective forearm, and each of said compartments (10) is provided with an opening (11), configured for a hand and forearm introduction, preferably of circular shape and provided in a membrane (12) in elastomeric material applied on the wall of each compartment (10), so to provide sealing to the operator's forearm once this is introduced, and to adapt to the various sizes of different hands and forearms.

Preferably, said compartments (10) have cylindrical shape and the membrane (12) is positioned at one of the two bases of the cylinder, in particular at the upper base.

The two compartments (10) are positioned so that it is possible to introduce a hand inside each of said two compartments. Preferably, said compartments are parallel to each other and - without this being limiting for the aims of the invention - they are spaced at a distance between 20 and 60 cm.

Whether there is one compartment (10) or two, the device is configured to receive two hands of an operator inside the containment volume and to provide sealing around his forearms.

The device comprises also means for ozone production (2), configured to generate an ozone flow to be introduced inside said at least one compartment (10).

Preferably, the device comprises also means (3) for atmospheric oxygen concentration, configured to send an air flow enriched with oxygen to said means for ozone production (2).

The device comprises also an air discharge duct (15) from inside said at least one compartment (10) outwards, and preferably an activated carbon filter (5) for converting ozone in oxygen.

Preferably, the device comprises also an extraction pump (51), arranged on the air discharge duct (15) and configured to extract air from the device through said discharge duct and to convey it towards the activated carbon filter and so outwards.

The pump (51) is configured so to compensate the aeraulic resistance of the activated carbon filter, so that inside the compartments (10) of the device an ozone flux can be provided without the pressure thereinside increases to values greater than ambient pressure. In this way, when the operator extracts his hands from the compartments at the end of the sanitization operations there is no ozone release in the ambient from the openings (11). This is clearly an advantage, which allows to use the device also in closed spaces and with is made possible only by the provision of the extraction pump (51) together with the sealing between operator forearms and membranes (12).

In order to avoid possible contaminations of the operator during the whole execution of the washing operation, the device comprises also, at each opening (11), a lid (6) configured to close said opening (11) and to define a volume (116) contained between the outer portion of said membrane (12) and said lid (6). In other words, when the lid (6) is closed the membrane (12) is entirely contained inside the closed space defined by the compartment (10) and lid (6), so that when the ozonized air is introduced inside the compartment (10), it comes in contact to the membrane (12) from both sides, thus sanitizing also the outer side of the membrane.

Without this being limiting for the aims of the invention, the lid (6) can be associated to one or more vent tubes (61), configured to convey air from the lid to the activated carbon filter (5). The provision of vent tubes allows to avoid air stagnation in the space between membrane (12) and lid (6), and so it guarantees that the ozone flow fills the space between membrane and lid entirely.

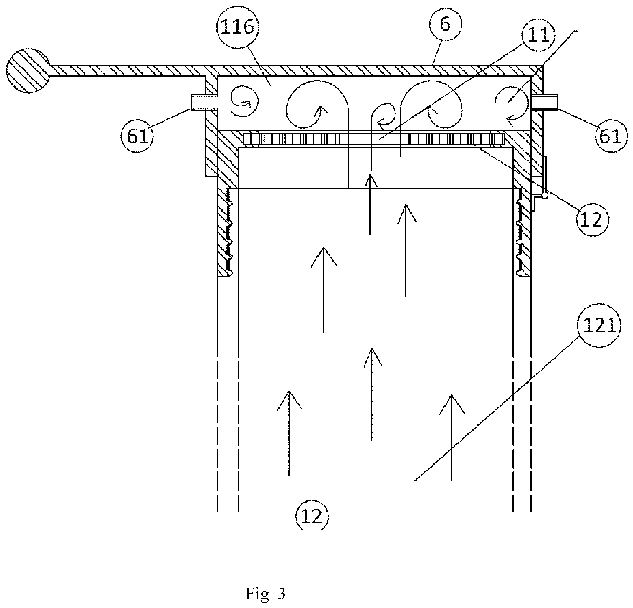

As it is shown in figures 1 and 3, the lid (6) is preferably hinged to the compartment (10), so to be opened and closed by means of a movement of rotation.

Preferably, the lid comprises also levers (66), with such dimensions that the opening and closing of the lid itself is allowed by means of the elbows, thus avoiding any hands contact with the device.

To such aim, as it is shown for example in figures 1 and 3, the lever (66) is arranged opposite to the hinge fastening the lid to the compartment, and projects to the outer wall of the compartment (10), so that the operator can open the lid by rotating it to its own hinge, simply by pushing the lever (66) upwards with the elbow, without needing grasping or blocking actions which would require to use hands.

The device according to the invention comprises also: means for detecting the opening or closing status of said lids, means for detecting the presence of the hands inside said at least one compartment, a timer and electronic control means, configured to receive signals from said detecting means and said timer and to control the ozone supply inside said at least one compartment, means for communication with the user, of acoustic or visual kind.

According to an embodiment, said means for detecting hands presence comprise one or more photoelectric cells.

On said electronic control means there are loaded computer programs configured:
- (100) to receive from the means for detecting hands presence a signal indicating that the operator's hands are introduced inside said at least one compartment (10);
- (200) to start the ozone supply inside said at least one compartment:
- (300) to end the ozone supply when said timer signals that a predetermined time interval is elapsed;
- (400) to communicate the end of the washing procedure to the user by means of said communication means.

Said electronic control means are also configured:
- (500) to start the ozone supply upon receiving from said means for detecting the lids condition a signal indicating that said lids are in closing condition;
- (600) to end the ozone supply when said timer signals that a predetermined time interval is elapsed;
- (700) to communicate the end of the sterilization procedure of the device to the user by means of said communication means.

Preferably, the device comprises also means for user identification and remote communication means. According to a preferred embodiment, said user identification means comprise a TAG RFID reader.

In this embodiment, said electronic control means are also configured to carry out, after step (100), the step of
- (150) receiving from the identification means a signal identifying an operator near the TAG RFID reader;
   and to carry out, after step (400), the step of
- (450) sending to a remote server the indication of date, time and identification of the user who used the device.

It is clear that, to such aim, each operator is preventively associated to a TAG RFID, which the same operator brings along.

It is to be specified now the utility of the shape of the lid (6) previously described.

After each usage of the device by an operator, the lid (6) can be closed and the ozone production means can be actuated, so to send ozonized air inside the compartment (10).

The ozonized air fills the whole inner space of the compartment (10), as well as the space closed by the compartment (10) and lid (6), and in particular it comes in contact to the membrane (12) from both sides.

When the lid (6) is opened before a new usage, the membrane (6) is sterilized also outside, and so there are no possibilities that the hand of the operator is contaminated in the extraction step from the device itself after sterilization.

The device, as shown in figure 1, comprises also a support (4) configured to hold said at least one compartment (10) in such a position to allow the introduction of the hands by the operator. Preferably, but not limitingly, the support allows the height and/or inclination adjustment of said compartments (10).

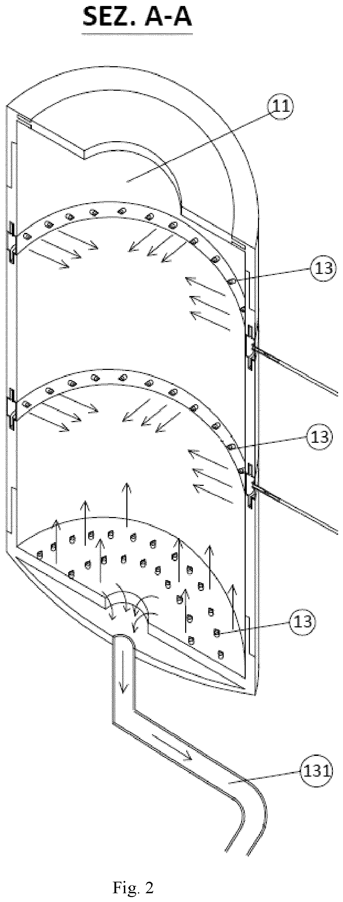

Preferably, as shown in figure 2, each compartment (10) comprises also a plurality of nozzles (13), arranged on the walls of the device and configured to spray water jets in the compartment (10). In case the compartment is cylindrical, the nozzles (13) are arranged both on the outer surface of the cylinder and on the base opposite to the one where the opening (11) is provided.

To such aim, the device comprises preferably a reservoir configured to contain demineralized water, and water pushing means to pump it through nozzles; as an alternative the device comprises connection means to the water supply network.

Reservoir, pushing means and connection means to water supply network are known per sé at the state of the art and they are not described in detail here.

In this embodiment, the device is configured to carry out, instead of step (200), the step of
- (200') starting water and ozone supply inside said at least one compartment.

Without this being limiting for the aims of the invention, the device comprises also pedal control means (7) configured to activate water and/or ozone supply inside said compartments (10).

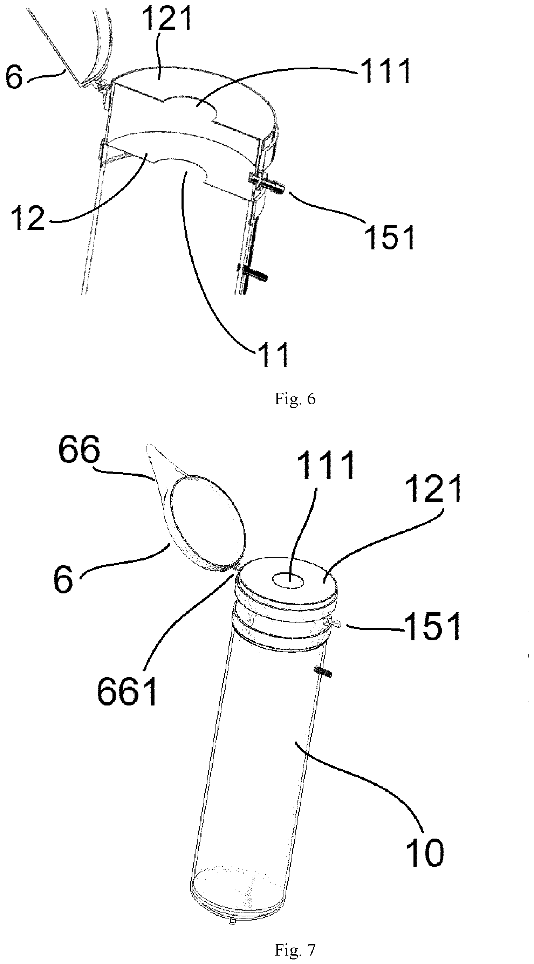

In another embodiment, shown in figure 6, each compartment (10) of the device comprises, in addition to the just described membrane (12), a second membrane (121) in elastomeric material, provided with a hole (111) as well. The two membranes (12, 121) are positioned spaced to each other, so that the two holes (11, 111) are substantially aligned, so that the operator's forearm, when the hand is introduced inside the compartment (10), seals with both the membranes.

On the wall of the compartment (10), in the space between the two membranes (12, 121) it is provided another vent (151), to which a duct (not shown in figure) is connected which connects said vent to the extraction pump (51), and so, to the activated carbons.

Since the membranes are made in elastomeric material (usually in silicone) and so they are elastic, in case of using only one membrane the movement of the operator's arm can cause a reduction in sealing of the first membrane, with possible ozone leakage. The usage of a second membrane according to what just explained, which, together with the first membrane, provides a sealing chamber with the forearm provided with suction means, reduces the probability that the limb movement ovalizes both the membranes with consequent ozone leakage.

## Claims

1. Device for hands sanitization (1), comprising:
- at least a compartment (10), with dimensions and shape suitable to receive the hands and at least a portion of forearms of an operator, said at least one compartment (10) being provided with a couple of openings (11) each configured for the introduction of a hand or forearm, provided in a membrane (12) of elastomeric material,
- means for ozone production (2), configured to generate an ozone flow to be introduced inside said at least one compartment (10);
- an air discharge duct (15) from inside said at least one compartment (10) outwards,
said device further comprising, at each of said openings (11), a respective lid (6),
**characterized in that** it comprises also:
- means for detecting the opening or closing status of said lids (6),
- means for detecting the presence of the hands inside each said compartments,
- a timer,
- means for communication with the user,
- electronic control means, configured to receive signals from said detecting means and said timer and to control the ozone supply inside said at least one compartment,
on said electronic control means being loaded computer programs, configured:
(100) to receive from the means for detecting hands presence a signal indicating that the operator's hands are introduced inside said at least one compartment (10);
(200) to start the ozone supply inside said at least one compartment;
(300) to end the ozone supply when said timer signals that a predetermined time interval is elapsed;
(400) to communicate the end of the washing procedure to the user by means of said communication means;
(500) to start the ozone supply upon receiving from said means for detecting the lids condition a signal indicating that said lids (6) are in closing condition;
(600) to end the ozone supply when said timer signals that a predetermined time interval is elapsed;
(700) to communicate the end of the sterilization procedure of the device to the user by means of said communication means.

2. Device for hands sanitization (1) according to claim 1, comprising two compartments (10), with dimensions and shape suitable to receive a hand and at least a portion of the respective forearm of an operator, each of said compartments being provided with an opening (11) configured for a hand and forearm introduction, provided in a membrane (12) in elastomeric material.

3. Device for hands sanitization (1) according to one of the preceding claims, further comprising means (3) for atmospheric oxygen concentration, configured to send an air flow enriched with oxygen to said means for ozone production (2).

4. Device for hands sanitization (1) according to one of the preceding claims, **characterized in that** to said discharge duct (15) an activated carbon filter (5) is associated for the conversion of ozone in oxygen, **in that** it comprises also an extraction pump (51) configured to extract air from the device through said discharge duct (15) and to convey it towards the activated carbon filter (5) (5) and so outwards.

5. Device for hands sanitization (1) according to one of the preceding claims, **characterized in that** each of said lids (6) is configured to close the respective opening (11) and to define a volume (116) contained between the outer portion of said membrane (12) and said lid (6), and **in that** to each of said lids (6) at least a vent tube (61) is associated, configured to convey air from said volume (116) towards said activated carbon filter (5).

6. Device for hands sanitization (1) according to one of the preceding claims, **characterized in that** each of said lids is hinged to the respective compartment (10) so to be opened and closed by means of a movement of rotation and **in that** each of said lids comprises a lever (66) arranged opposite to the hinge fastening each lid (6) to the respective compartment (10), said lever being projecting to the outer wall of the compartment (10), so that the operator can open the lid by rotating it to its own hinge, simply by pushing the lever (66) upwards with the elbow.

7. Device for hands sanitization (1) according to one of the preceding claims, comprising also means for user identification and remote communication means, and **characterized in that** said electronic control means are configured to carry out, after step (100), the step of
(150) receiving from the identification means a signal identifying an operator near the device; and to carry out, after step (400), the step of
(450) sending to a remote server the indication of date, time and identification of said operator near the device.

8. Device for hands sanitization (1) according to one of the preceding claims, **characterized in that** each compartment (10) comprises a plurality of nozzles (13), arranged on the walls of the device and configured to spray water jets in the compartment (10) and **in that** the device is configured to carry out, instead of step (200), the step of
- (200') starting water and ozone supply inside said at least one compartment.

9. Device for hands sanitization (1) according to claim 8, further comprising a reservoir configured to contain demineralized water and water pushing means configured to pump it through said nozzles.

10. Device for hands sanitization (1) according to one of the preceding claims, **characterized in that** each compartment (10) comprises a second membrane (121) in elastomeric material, provided with a hole (111) as well, the two membranes (12, 121) being positioned spaced to each other, so that the two holes (11, 111) are substantially aligned, so that the operator's forearm, when the hand is introduced inside the compartment (10), seals with both the membranes.

11. Device for hands sanitization (1) according to claim 10, further comprising on the wall of the compartment (10), in the space between the two membranes (12, 121), another vent (151), to which a duct is associated which connects said vent to said extraction pump (51).
